# EUROPEAN PATENT SPECIFICATION

(11) **EP 3 842 016 B1**
(45) Date of publication and mention of the grant of the patent: **28.08.2024**
(21) Application number: 19219715.0
(22) Date of filing: 25.12.2019
(51) Int. Cl.: A61F 13/49, A61F 13/491

(54) **MALE INCONTINENCE ARTICLE**
INKONTINENZARTIKEL FÜR MÄNNER
ARTICLE POUR INCONTINENCE MASCULINE

(43) Date of publication of application: 30.06.2021
(73) Proprietor: Ontex BV, 9255 Buggenhout (BE); Ontex Group NV, 9320 Erembodegem (BE)
(72) Inventor: Montepara, Paola, 66036 Orsogna (IT)
(74) Representative: Larangé, Françoise

(56) References cited:
- EP-A1- 1 754 461
- EP-A1- 2 554 149
- WO-A1-97/15260
- WO-A1-99/33422
- US-A1- 2019 099 304

## Description

### TECHNICAL FIELD

The disclosure pertains to the technical field of absorbent personal hygiene products and in particular disposable absorbent products such as male incontinence absorbent articles.

### BACKGROUND

Absorbent articles for personal hygiene such as disposable diapers, feminine protection pads and adult incontinence undergarments, are designed to absorb and contain body exudates, in particular but not limited to urine. These absorbent articles usually comprise several layers having different functions, for example a topsheet, a backsheet and in-between an absorbent core, among other layers. The absorbent core's function is to absorb and retain the exudates for a prolonged amount of time, for example overnight for a diaper, minimize re-wet to keep the wearer dry and avoid soiling of clothes or bed sheets.

Absorbent cores can expand several times their initial volumes when wet. It is desirable that the cores in this expanded state maintain their structural integrity and do not break or burst even when submitted to a shock such as a child sitting heavily on his diaper. It is generally also desirable that absorbent cores should be thin (at least when dry) and require as little material as possible for costs and environmental reasons.

Absorbent incontinence articles for male as known in the art typically comprise a combination of elastified sections in a waist region and an absorbent core in a crotch region.

For example, EP1549272 B1 discloses a pants-shaped absorbent product, such as incontinence pants or pull-ups, intended for males, comprising a front section, a rear section, a crotch section , an elastic waist region, and an absorbent element intended to cover the genitalia of the user, and further comprising a liquid-tight outer layer. The front section has an elastic member, which, during product usage, enables the front section of the product to be pulled down, counter to the action of the said elastic member, to a position in which the upper limit edge of the front section is situated below the penis of the user. The absorbent element is configured with one or more deformation zones, which enable parts of the absorbent element to be drawn along when the front section of the product is pulled down. The front section of the product, together with the absorbent element, are arranged to be returned by the elastic member to their original usage position. Such articles however have drawbacks with respect to breathability and overall comfort over time, as well as leakage on the waist region during incontinence gushes.

Further improvements in male incontinence articles are described for example in WO2018/182469 which relates to a disposable pant-type absorbent article, such as a pant diaper, a sanitary pant or incontinence pant being adapted for a male user, and a method for manufacturing such a disposable pant-type absorbent article. The disposable pant-type absorbent article is characterised in that the absorbent core of the article has a wide portion in the front segment. The article is further characterised in that the laminated web material of the front panel of the article has a first at least partly elastic region extending primarily in the transverse direction and being located adjacent the leg edges of the front panel, and a second at least partly elastic region extending primarily in the transverse direction and being located next to the first elastic region and closer to the waist edge of the front panel. The first at least partly elastic region is distinct from the second at least partly elastic region in terms of at least one structural elastic feature. An elasticised part of the laminated web material of the first at least partly elastic region or an inner imaginary extension of the elasticised part meet a longitudinal core edge in said wide portion of the core. Whilst problems such as improved breathability and comfort may be addressed in a cost-effective manner, drawbacks such as leakage risk towards the front waist region still exist as well as still a need for better support to the male genitalia in still a cost-effective manner.

EP1754461 discloses a disposable wearing article including a partition which in use is spaced upward from the topsheet. During use the wearer's genital organ is also spaced from the topsheet and in this way, protected from soiling. EP2554149 discloses a disposable wearing article wherein the crotch panel may be stably fastened to the outer surface of the waist panel. WO99/33422 and US20190099304 describe absorbent articles adapted for the adult male anatomy and WO97/15260 relates to an article which incorporates a distinctively elasticized barrier or containment system at a waistband portion of the article.

There thus still remains a need to provide disposable incontinence diapers that are specifically designed for male subjects and that significantly improves comfort and leakage prevention especially over the front waist thereof, whilst still being cost-effective.

### SUMMARY OF THE INVENTION

In a first aspect, the disclosure relates to a male incontinence disposable absorbent article comprising: a waistband assembly configured to be worn about a waist of a subject; and an absorbent panel assembly having first and second ends that are coupled to the waistband assembly, the absorbent panel assembly comprising a liquid-pervious topsheet, a liquid impervious backsheet, and an absorbent core positioned between the topsheet and backsheet, and further comprising lateral cuffs extending along a length of said core and joined to at least a portion of said topsheet on a skin-facing surface thereof; wherein the absorbent panel assembly is arranges such that it overlaps at least a portion of the waistband assembly in at least a front and/or back portion thereof, and wherein the waistband assembly comprises a first waist region, a second waist region, and a third waist region, each said waist regions comprising a plurality of elastic strands arranged to provide a contraction force in a transverse direction, wherein said elastic strands of said first waist region have a first spacing, said elastic strands of said second waist region have a second spacing, and said elastic strands of said third waist region have a third spacing, wherein said absorbent panel assembly comprises a frontal barrier comprising a nonwoven substrate that is joined to at least portions of each said lateral cuffs at distinct joining zones and wherein said joining zones do not substantially overlap with the topsheet in areas thereof that are free from said lateral cuffs such that a pocket is formed, and wherein said nonwoven substrate is joined to said topsheet along a further joining zone that is positioned between said distinct joining zones and along an extremity of the pocket along a transverse direction crossing the distinct joining zones, and in that said pocket is arranged to provide for containment of the male genitalia when the article is worn by a subject and/or provide a barrier to leakage on the front of said article towards the waistband assembly, wherein the second waist region is positioned between the third waist region and a the first waist region and wherein the first spacing is less than the second and third spacings, and wherein the article comprises a contraction region that extends from the first waist region, and/or second waist region, to the third waist region and overlaps at least a portion of the absorbent panel assembly, preferably wherein the contraction region comprises one or more curved elastics arranged to provide a contraction force from the absorbent panel assembly towards the first and/or second waist regions.

### DESCRIPTION OF FIGURES

Fig. 1 illustrates a schematic of an article according to an embodiment of the disclosure.
Fig. 2 illustrates a schematic of an article according to an embodiment of the disclosure.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-5% or less, preferably +/-1% or less, and more preferably +/-0.1 % or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation or element referred to.

The recitation of numerical ranges by endpoints includes all numbers and fractions subsumed within that range, as well as the recited endpoints, except where otherwise explicitly stated by disclaimer and the like.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious top sheet, a back sheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

The "absorbent medium" or "absorbent core" or "absorbent body" is the absorbent structure disposed between the top sheet and the back sheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface of a film layer in the required pattern or network of adhesive areas to form the film-nonwoven laminate of the present invention. Accordingly, suitable adhesives include conventional hot melt adhesives, pressure-sensitive adhesives and reactive adhesives (i.e., polyurethane).

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used herein, the term "associated or joined or adhered" encompasses, unless expressly stated, configurations in which e.g. a top sheet is directly joined to a back sheet by affixing the top sheet directly to the back sheet, and also configurations wherein the top sheet is joined to the back sheet by affixing the top sheet to intermediate members which in turn are affixed to the back sheet (i.e. indirectly joining). Top sheet and back sheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix top sheet to back sheet. It should be readily appreciated that the above-described attachment means may also be employed to interconnect and assemble together the various other component parts of the article described herein.

The terms "back portion", "back section" and "rear back section" are used herein as synonyms and refer to the area of the absorbent article which is contact with the back of the wearer when the absorbent article is worn.

The term "backsheet" refers to a material forming a liquid impermeable cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated.

The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The terms "front portion", "belly section" and "front belly section" are used herein as synonyms and refer to the area of the absorbent article which is contact with the belly of the wearer when the absorbent article is worn.

As used herein, the "body-facing" or "bodyside" or "skin-facing" surface means that surface of the article or component which is intended to be disposed toward or placed adjacent to the body of the wearer during ordinary use, while the "outward", "outward-facing", "garment-facing" or "garment-side" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

Further, an absorbent article can comprise "containment flaps" or "barrier cuffs". The containment flaps are generally thought to be particularly well suited for the containment of fecal matter and to prevent the lateral flow of liquid waste until such time as the liquid waste can be absorbed by the absorbent article. Many constructions of containment flaps are known. Such containment flaps generally comprise a proximal edge, intended to be attached to the absorbent article, and an opposite distal edge which is generally not attached to the absorbent article along at least a portion of its length. An elastic member is generally located adjacent the distal edge to assist in maintaining the containment flap in an upright condition and in maintaining a sealing relationship between the distal edge of the containment flap and the body of a wearer during use. The elastic member is generally located between two layers of material so that the elastic does not come into contact with the body of a wearer. The containment flaps may be manufactured from a wide variety of materials such as polypropylene, polyester, rayon, nylon, foams, plastic films, formed films, and elastic foams. A number of manufacturing techniques may be used to manufacture the containment flaps. For example, the containment flaps may be woven, non-woven, spunbonded, carded, cast, blown or the like.

An absorbent article can comprise leg containment gaskets (also broadly referred to as "cuffs"). Leg "containment gaskets" help prevent leakage of bodily exudates when the wearer exerts compressive forces on the absorbent article. In particular, the stiffness of the leg containment gaskets prevents twisting and bunching of the leg openings of the absorbent article which can lead to leaks. In addition, the elasticity and conformability of the leg containment gaskets ensures that the bodyfacing surface of the leg containment gaskets provides an adequate seal against the body of the wearer. The physical properties of the leg containment gaskets, such as the thickness and stiffness, also function to space the bodyside liner, outer cover and absorbent core away from the wearer's body when in use. As such, void volume is created between the wearer's body and the bodyside liner and absorbent core of the absorbent article to help contain body exudates.

"Mechanical bond" is an attachment between two or more elements, components, regions, or webs and may comprise heat bonds, pressure bonds, ultrasonic bonds, dynamic mechanical bonds, or any other suitable non-adhesive attachment means or combinations of these attachment means.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The term "laid-flat state" or "fully stretched state" or "extended state" is intended to refer to the article when it is flattened into a plane or is substantially flattened into a plane and is used in contrast to when the article otherwise positioned, such as when the article is folded or shaped in or for use by a wearer.

The use of the term "layer" can refer, but is not limited, to any type of substrate, such as a woven web, nonwoven web, films, laminates, composites, elastomeric materials, or the like, or even formulations, such as adhesives, that form a substrate upon a change in conditions (e.g. solidification of a hotmelt adhesive when the temperature drops below a predetermined amount). A layer can be liquid and air permeable, permeable to air but impermeable to liquids, impermeable both to air and liquid, or the like. When used in the singular, it can have the dual meaning of a single element or a plurality of elements.

The term "nonwoven substrate/fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

Use of the term "substrate" includes, but is not limited to, woven or nonwoven webs, porous films, ink permeable films, paper, composite structures, or the like.

Superabsorbent materials (e.g. superabsorbent polymers) suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present invention in an amount up to 100% by weight. Typically, the superabsorbent material, when present, will be included in an amount of from about 30% to about 70% by weight, based on the total weight of the absorbent layer.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

The term "topsheet" refers to a liquid permeable material sheet forming the inner cover of the absorbent article and which in use is placed in direct contact with the skin of the wearer. The topsheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

As used herein, the term "transverse" or "lateral" refers to a line, axis, or direction which lies within the plane of the absorbent article and is generally perpendicular to the longitudinal direction (i.e. is parallel to the x-axis).

The term "spunbond fibers" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 5-60 µm, preferably from 10-30 µm. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated in the SMS fabric, for example spunbond-meltblown-meltblown-spunbond (SMMS), etc.

Embodiments according to the disclosure will now be described. It is understood that technical features described in one or more embodiments maybe combined with one or more other embodiments without departing from the intention of the disclosure and without generalization therefrom.

### THE ABSORBENT ARTICLE

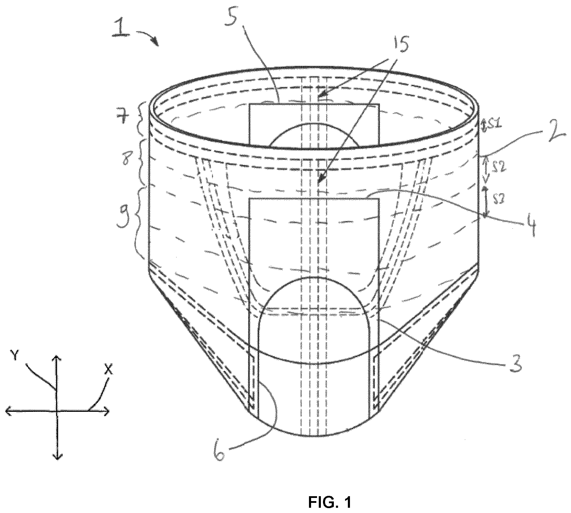

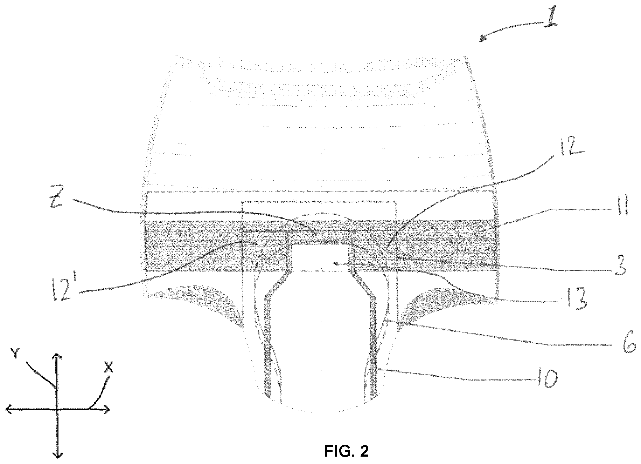

As exemplified in Figs. 1 and 2, the disclosure relates to male incontinence disposable absorbent article (1) comprising: a waistband assembly (2) configured to be worn about a waist of a subject; and an absorbent panel assembly (3) having first and second ends (4, 5) that are coupled to the waistband assembly (2), the absorbent panel assembly comprising a liquid-pervious topsheet, a liquid impervious backsheet, and an absorbent core (6) positioned between the topsheet and backsheet, and further comprising lateral cuffs (10) extending along a length of said core (6) and joined to at least a portion of said topsheet on a skin-facing surface thereof; wherein the absorbent panel assembly (3) is arranges such that it overlaps at least a portion of the waistband assembly (2) in at least a front and/or back portion thereof, and wherein the waistband assembly (2) comprises a first waist region (7), a second waist region (8), and a third waist region (9), each said waist regions (7, 8, 9) comprising a plurality of elastic strands arranged to provide a contraction force in a transverse direction (i.e. a direction substantially perpendicular to the longitudinal axis y), wherein said elastic strands of said first waist region (7) have a first spacing (s1), said elastic strands of said second waist region (8) have a second spacing (s2), and said elastic strands of said third waist region (9) have a third spacing (s3), wherein said absorbent panel assembly (3) comprises a frontal barrier (11), typically positioned on the front portion of the article (1), comprising a nonwoven substrate that is transversely joined (typically such that the barrier extends continuously along a transverse direction running substantially parallel to the axis x) to at least portions of each said lateral cuffs (10) at distinct joining zones (12, 12') and wherein said joining zones (12, 12') do not substantially directly engage with the topsheet in areas thereof that are free from said lateral cuffs (10) such that a pocket (13) is formed, and wherein said nonwoven substrate is joined to said topsheet along a further joining zone (Z) that is positioned between said distinct joining zones (12, 12') and along an extremity of the pocket (13) along a transverse direction crossing the distinct joining zones (12, 12'), and in that said pocket (13) is arranged to provide for containment of the male genitalia when the article is worn by a subject and/or provide a barrier to leakage on the front of said article (1) towards the waistband assembly (2). Advantageously the combination of a pocket as described and the overlapping elastic strands arrangement described not only provides for a breathable product (compared to the use of film elastic webs) but further improves the comfort and support to the male genitalia and additionally leakage prevention.

The waistband assembly (2) may comprise a front panel (generally positioned at a front portion of the article (1)) and a back panel (generally positioned at a back portion of the article (1)). The front and back panels are typically disposed at opposite ends, when the article is in laid-flat/extended state, with the absorbent panel assembly (3) disposed therebetween, and wherein the front and back panel are joined together at lateral seams to form a pant-like article comprising a waist opening formed by the uppermost edge of the front and back panels and two leg openings formed by the lowermost edges of the front and back panels and lateral side edges of the absorbent panel assembly (3). Each said panel may comprise an outer sheet laminated to an inner sheet with the elastic strands sandwiched therebetween. The elastic strands may be glued to the outer and inner sheets via strand-coating (i.e. coating of the elastics with adhesive such that substantially no adhesive is present in gaps between elastic strands) for improved softness and reduced usage of adhesive and at least the first waist region (7) comprises further auxiliary adhesive in a spacing between elastic strands to further secure terminal edges of the waist region. The inner and outer sheets may be nonwoven substrates, typically laminates comprising spunbond and meltblown nonwoven layers such as an SMS or SMMS nonwoven laminates.

Preferably, the nonwoven substrate is hydrophobic or hydrophobically treated for example with a surfactant coating. Typically this substrate is a spunbond nonwoven or a laminate of spunbond and meltblown nonwoven layers such as an SMS or SMMS nonwoven. The advantage of spunbonds or laminates comprising spunbond outermost layers is to provide softness and thus comfort to the wearer, on the otherhand the meltblone inner layers provide for added strength and tear resistance to the nonwoven.

Preferably, the nonwoven substrate has a basis weight of from 10 to 50 g/m², preferably from 12 to 25 g/m², more preferably from 16 to 20 g/m². Advantageously, this range allows for sufficient structural integrity and resistance to tear whilst being soft to the touch and limiting cost.

The second waist region (8) is positioned between the third waist region (9) and a the first waist region (7) and wherein the first spacing is less than the second and third spacings, and wherein the article (1) comprises a contraction region that extends from the first waist region (7), and/or second waist region (8), to the third waist region (9) and overlaps at least a portion of the absorbent panel assembly (3), preferably wherein the contraction region comprises one or more curved elastics arranged to provide a contraction force from the absorbent panel assembly (3) towards the first and/or second waist regions (7, 8). Such allows for graded stretch properties that allow good support to in the crotch region with the absorbent assembly being pulled up by contraction forces towards the waist regions.

In a preferred embodiment, the contraction region overlaps the pocket (13) and an apex (15) of said contraction region extends beyond said pocket (13) in a longitudinal direction that crosses the waistband assembly (2) and the absorbent panel assembly (3). Advantageously this provides for added support to the genitalia as well as creating a containment void region that helps prevent leakage in the waist region.

Preferably, the nonwoven substrate is arranged to extend laterally beyond the absorbent panel assembly (3) and into the third waist region (9). Such arrangement allows for better structural integrity and resistance to tear. Advantageously mechanical bonding may be used in these outboard regions to provide for added comfort over time.

Preferably, the pocket (13) comprises a flap that forms an opening of said pocket (13) together with a skin-facing surface of the topsheet. Typically, at least the flap comprises indicia, preferably being a colour, such that the flap may be recognised and lifted for containment of the male genitalia when worn by a subject.

The flap may further comprise one or more elastic strands, preferably one or more flat-elastics, at the apex thereof (similar to what know in lateral cuffs in the art that rather use Lycra strands having circular cross-section) and this may help the flap to more easily be grasped and stand up at a distance with respect to a skin-facing surface of the topsheet.

In an embodiment, the third spacing is greater than the first and second spacings. This allows for reduced elasticity in the region of the genitalia such to limit discomfort whilst still providing some support which is also desirable to render the leakage prevention of the pocket effective.

The first and second waist regions (7,8) typically comprise straight elastics and the third region (9) comprises curved elastics. Advantageously this improves the comfort of the product.

Preferably, the first and second waist regions (7, 8) do not overlap the absorbent panel assembly (3).

Preferably the contraction region is positioned on the front of the article (1), preferably only said front. Advantageously this provides for a vertical contraction force in a direction substantially parallel to the longitudinal axis y (that runs through the front waistband assembly, absorbent panel assembly, and back waistband assembly respectively) that provides the required support where needed.

In an embodiment, the contraction region is positioned on the front and the back of the article (1), with the proviso that the in the back, the contraction region extends closer to a transverse centreline (this being the centreline of the absorbent panel assembly taken along an axis perpendicular to the longitudinal axis y - typically when looking at it in a laid-flat state) of the absorbent panel assembly (3) compared to in the back. Advantageously and counter-intuitively it has been found that this arrangement not only allows for both front and back portions of the article to be pulled up towards the waist but further comfort of the male genitalia as well as leakage prevention aid is enhanced compared to when the contraction regions extend at the same length or with the front extending further to the crotch than the back.

In a preferred embodiment, articles herein comprise a further contraction region (15) continuously extending from the first and/or second waist regions (7, 8) in the front of said article (1) to the first and/or second waist regions (7, 8) in the back of said article (1) and overlapping the entire absorbent panel assembly (3). The further contraction region may comprise one or more elastic strands that are preferably linear, and most preferably are so-called flat-elastics (i.e. have a substantially line-form cross-section compared to classic strand elastics like Lycra that have substantially circular cross-section). Advantageously, this arrangement allows for general support of the crotch region through the entire absorbent panel assembly that is held towards the waist opening.

It is supposed that the present invention is not restricted to any form of realization described previously and that some modifications can be added to the presented example of fabrication without reappraisal of the appended claims.

## Claims

1. A male incontinence disposable absorbent article (1) comprising:
a waistband assembly (2) configured to be worn about a waist of a subject; and
an absorbent panel assembly (3) having first and second ends (4, 5) that are coupled to the waistband assembly (2), the absorbent panel assembly comprising a liquid-pervious topsheet, a liquid impervious backsheet, and an absorbent core (6) positioned between the topsheet and backsheet, and further comprising lateral cuffs (10) extending along a length of said core (6) and joined to at least a portion of said topsheet on a skin-facing surface thereof;
wherein the absorbent panel assembly (3) is arranged such that it overlaps at least a portion of the waistband assembly (2) in at least a front and/or back portion thereof, and wherein the waistband assembly (2) comprises a first waist region (7), a second waist region (8), and a third waist region (9), each said waist regions (7, 8, 9) comprising a plurality of elastic strands arranged to provide a contraction force in a transverse direction, wherein said elastic strands of said first waist region (7) have a first spacing (s1), said elastic strands of said second waist region (8) have a second spacing (s2), and said elastic strands of said third waist region (9) have a third spacing (s3), **characterised in that** said absorbent panel assembly (3) comprises a frontal barrier (11) comprising a nonwoven substrate that is transversely joined to at least portions of each said lateral cuffs (10) at distinct joining zones (12, 12') and wherein said joining zones (12, 12') do not substantially directly engage with the topsheet in areas thereof that are free from said lateral cuffs (10) such that a pocket (13) is formed, and wherein said nonwoven substrate is joined to said topsheet along a further joining zone (Z) that is positioned between said distinct joining zones (12, 12') and along an extremity of the pocket (13) along a transverse direction crossing the distinct joining zones (12, 12'), and **in that** said pocket (13) is arranged to provide for containment of the male genitalia when the article is worn by a subject and/or provide a barrier to leakage on a front portion of said article (1) towards the waistband assembly (2), wherein the second waist region (8) is positioned between the third waist region (9) and a the first waist region (7) and wherein the first spacing is less than the second and third spacings, and wherein the article (1) comprises a contraction region that extends from the first waist region (7), and/or second waist region (8), to the third waist region (9) and overlaps at least a portion of the absorbent panel assembly (3), preferably wherein the contraction region comprises one or more curved elastics arranged to provide a contraction force from the absorbent panel assembly (3) towards the first and/or second waist regions (7, 8).

2. An article (1) according to Claim 1 wherein the nonwoven substrate is hydrophobic or hydrophobically treated.

3. An article (1) according to Claim 1 or 2 wherein the contraction region overlaps the pocket (13) and an apex (15) of said contraction region extends beyond said pocket (13) in a longitudinal direction that crosses the waistband assembly (2) and the absorbent panel assembly (3).

4. An article (1) according to any of the preceding Claims wherein the nonwoven substrate is arranged to extend laterally beyond the absorbent panel assembly (3) and into the third waist region (9).

5. An article (1) according to any of the preceding Claims wherein the pocket (13) comprises a flap that forms an opening of said pocket (13) together with a skin-facing surface of the topsheet.

6. An article (1) according to Claim 5 wherein at least the flap comprises indicia, preferably being a colour, such that the flap may be recognised and lifted for containment of the male genitalia when worn by a subject.

7. An article (1) according to Claim 1 wherein the third spacing is greater than the first and second spacings.

8. An article (1) according to any of the preceding Claims wherein the first and second waist regions (7, 8) do not overlap the absorbent panel assembly (3).

9. An article (1) according to any of the preceding Claims wherein the contraction region is positioned on the front of the article (1), preferably only said front.

10. An article (1) according to any of the preceding Claims wherein the contraction region is positioned on the front and the back of the article (1), with the proviso that the in the front, the contraction region extends closer to a transverse centreline of the absorbent panel assembly (3) compared to in the back.

11. An article (1) according to any of the preceding Claims being in the form of a pant having side seams that connect front to back portions of the waistband assembly (2) together.

12. An article (1) according to any of the previous Claims comprising a further contraction region (15) continuously extending from the first and/or second waist regions (7, 8) in the front of said article (1) to the first and/or second waist regions (7, 8) in the back of said article (1) and overlapping the entire absorbent panel assembly (3).

## Patentansprüche

1. Saugfähiger Einweg-Inkontinenzartikel (1) für Männer, umfassend:
eine Taillenbundanordnung (2), die konfiguriert ist, um um eine Taille einer Person getragen zu werden; und
eine saugfähige Panelanordnung (3), die erste und zweite Enden (4, 5) aufweist, die mit der Taillenbundanordnung (2) gekoppelt sind, wobei die saugfähige Panelanordnung eine flüssigkeitsdurchlässige Oberschicht, eine flüssigkeitsundurchlässige Unterschicht und einen zwischen der Oberschicht und Unterschicht angeordneten saugfähigen Kern (6) umfasst und weiter seitliche Bündchen (10) umfasst, die sich entlang einer Länge des Kerns (6) erstrecken und mit zumindest einem Abschnitt der Oberschicht auf einer der Haut zugewandten Oberfläche davon verbunden sind;
wobei die saugfähige Panelanordnung (3) angeordnet ist, sodass sie sich mit zumindest einem Abschnitt der Taillenbundanordnung (2) in zumindest einem vorderen und/oder hinteren Abschnitt davon überlappt, und wobei die Taillenbundanordnung (2) einen ersten Taillenbereich (7), einen zweiten Taillenbereich (8) und einen dritten Taillenbereich (9) umfasst, wobei jeder der Taillenbereiche (7, 8, 9) eine Vielzahl von elastischen Strängen umfasst, die angeordnet sind, um eine Kontraktionskraft in einer Querrichtung bereitzustellen, wobei die elastischen Stränge des ersten Taillenbereichs (7) einen ersten Abstand (s1) aufweisen, die elastischen Stränge des zweiten Taillenbereichs (8) einen zweiten Abstand (s2) aufweisen und die elastischen Stränge des dritten Taillenbereichs (9) einen dritten Abstand (s3) aufweisen, **dadurch gekennzeichnet, dass die** saugfähige Panelanordnung (3) eine vordere Barriere (11) umfasst, die ein Vliessubstrat umfasst, das quer mit zumindest Abschnitten von jedem seitlichen Bündchen (10) in unterschiedlichen Verbindungszonen (12, 12') verbunden ist, und wobei die Verbindungszonen (12, 12') in Bereichen davon, die frei von den seitlichen Bündchen (10) sind, im Wesentlichen nicht direkt mit der Oberschicht in Eingriff stehen, sodass eine Tasche (13) gebildet wird, und wobei das Vliessubstrat mit der Oberschicht entlang einer weiteren Verbindungszone (Z) verbunden ist, die zwischen den unterschiedlichen Verbindungszonen (12, 12') und entlang eines Endes der Tasche (13) entlang einer Querrichtung angeordnet ist, welche die unterschiedlichen Verbindungszonen (12, 12') kreuzt, und dadurch, dass die Tasche (13) angeordnet ist, um einen Einschluss der männlichen Genitalien bereitzustellen, wenn der Artikel von einer Person getragen wird und/oder um eine Barriere gegen Auslaufen an einem vorderen Abschnitt des Artikels (1) in Richtung der Taillenbundanordnung (2) bereitzustellen, wobei der zweite Taillenbereich (8) zwischen dem dritten Taillenbereich (9) und dem ersten Taillenbereich (7) positioniert ist, und wobei der erste Abstand kleiner ist als der zweite und dritte Abstand, und wobei der Artikel (1) einen Kontraktionsbereich umfasst, der sich vom ersten Taillenbereich (7) und/oder zweiten Taillenbereich (8) zum dritten Taillenbereich (9) erstreckt und zumindest einen Abschnitt der saugfähigen Panelanordnung (3) überlappt, wobei der Kontraktionsbereich vorzugsweise ein oder mehrere gekrümmte Gummibänder umfasst, die angeordnet sind, um eine Kontraktionskraft von der saugfähigen Panelanordnung (3) in Richtung des ersten und/oder zweiten Taillenbereiches (7, 8) bereitzustellen.

2. Artikel (1) nach Anspruch 1, wobei das Vliessubstrat hydrophob ist oder hydrophob behandelt ist.

3. Artikel (1) nach Anspruch 1 oder 2, wobei der Kontraktionsbereich die Tasche (13) überlappt und sich ein Scheitelpunkt (15) des Kontraktionsbereichs über die Tasche (13) hinaus in einer Längsrichtung erstreckt, welche die Taillenbundanordnung (2) und die saugfähige Panelanordnung (3) kreuzt.

4. Artikel (1) nach einem der vorstehenden Ansprüche, wobei das Vliessubstrat angeordnet ist, um sich seitlich über die saugfähige Panelanordnung (3) hinaus und in den dritten Taillenbereich (9) hinein zu erstrecken.

5. Artikel (1) nach einem der vorstehenden Ansprüche, wobei die Tasche (13) eine Klappe umfasst, die zusammen mit einer der Haut zugewandten Oberfläche der Oberschicht eine Öffnung der Tasche (13) bildet.

6. Artikel (1) nach Anspruch 5, wobei zumindest die Klappe Kennzeichen, die vorzugsweise eine Farbe sind, umfasst, sodass die Klappe zum Einschließen der männlichen Genitalien erkannt und angehoben werden kann, wenn sie von einer Person getragen wird.

7. Artikel (1) nach Anspruch 1, wobei der dritte Abstand größer ist als der erste und zweite Abstand.

8. Artikel (1) nach einem der vorstehenden Ansprüche, wobei der erste und der zweite Taillenbereich (7, 8) die saugfähige Panelanordnung (3) nicht überlappen.

9. Artikel (1) nach einem der vorstehenden Ansprüche, wobei der Kontraktionsbereich auf der Vorderseite des Artikels (1), vorzugsweise nur auf dieser Vorderseite positioniert ist.

10. Artikel (1) nach einem der vorstehenden Ansprüche, wobei der Kontraktionsbereich auf der Vorderseite und der Rückseite des Artikels (1) mit der Maßgabe positioniert ist, dass sich der Kontraktionsbereich auf der Vorderseite näher an einer Quermittellinie der saugfähigen Panelanordnung (3) erstreckt als auf der Rückseite.

11. Artikel (1) nach einem der vorstehenden Ansprüche in Form einer Hose, die Seitennähte aufweist, die Abschnitte von vorne nach hinten der Hosenbundanordnung (2) miteinander verbindet.

12. Artikel (1) nach einem der vorstehenden Ansprüche, der einen weiteren Kontraktionsbereich (15) umfasst, der sich kontinuierlich von dem ersten und/oder zweiten Taillenbereich (7, 8) an der Vorderseite des Artikels (1) bis zum ersten und/oder zweiten Taillenbereich (7, 8) auf der Rückseite des Artikels (1), erstreckt und die gesamte saugfähige Panelanordnung (3) überlappt.

## Revendications

1. Article (1) absorbant jetable pour l'incontinence masculine comprenant :
un ensemble ceinture (2) configuré pour être porté autour de la taille d'un sujet ; et
un ensemble panneau absorbant (3) présentant des première et seconde extrémités (4, 5) qui sont couplées à l'ensemble ceinture (2), l'ensemble panneau absorbant comprenant une feuille supérieure perméable aux liquides, une feuille arrière imperméable aux liquides et une partie centrale absorbant (6) positionné entre la feuille supérieure et la feuille arrière, et comprenant en outre des revers latéraux (10) s'étendant sur une longueur de ladite partie centrale (6) et reliés à au moins une partie de ladite feuille supérieure sur une surface de celle-ci faisant face à la peau ;
dans lequel l'ensemble panneau absorbant (3) est agencé de telle sorte qu'il chevauche au moins une partie de l'ensemble ceinture (2) dans au moins une partie avant et/ou arrière de celui-ci, et dans lequel l'ensemble ceinture (2) comprend une première région de taille (7), une deuxième région de taille (8) et une troisième région de taille (9), chacune desdites régions de taille (7, 8, 9) comprenant une pluralité de rubans élastiques agencés pour fournir une force de contraction dans une direction transversale, dans lequel lesdits rubans élastiques de ladite première région de taille (7) présentent un premier espacement (s1), lesdits rubans élastiques de ladite deuxième région de taille (8) présentent un deuxième espacement (s2) et lesdits rubans élastiques de ladite troisième région de taille (9) présentent un troisième espacement (s3), **caractérisé en ce que** ledit ensemble panneau absorbant (3) comprend une barrière frontale (11) comprenant un substrat non tissé qui est relié transversalement à au moins des parties de chacun desdits revers latéraux (10) au niveau de zones de jonction (12, 12') distinctes et dans lequel lesdites zones de jonction (12, 12') n'entrent sensiblement pas directement en contact avec la feuille supérieure dans des zones de celle-ci qui sont dépourvues desdits revers latéraux (10) de telle sorte qu'une poche (13) soit formée, et dans lequel ledit substrat non tissé est relié à ladite feuille supérieure le long d'une autre zone de jonction (Z) qui est positionnée entre lesdites zones de jonction (12, 12') distinctes et le long d'une extrémité de la poche (13) le long d'une direction transversale coupant les zones de jonction (12, 12') distinctes, et **en ce que** ladite poche (13) est conçue pour assurer la retenue des organes génitaux masculins lorsque l'article est porté par un sujet et/ou fournir une barrière contre les fuites sur une partie avant dudit article (1) vers l'ensemble ceinture (2), dans lequel la deuxième région de taille (8) est positionnée entre la troisième région de taille (9) et la première région de taille (7) et dans lequel le premier espacement est inférieur aux deuxième et troisième espacements, et dans lequel l'article (1) comprend une région de contraction qui s'étend de la première région de taille (7), et/ou la deuxième région de taille (8), à la troisième région de taille (9) et chevauche au moins une partie de l'ensemble panneau absorbant (3), de préférence dans lequel la région de contraction comprend un ou plusieurs élastiques incurvés conçus pour fournir une force de contraction de l'ensemble panneau absorbant (3) vers la première et/ou la deuxième région(s) de taille (7, 8).

2. Article (1) selon la revendication 1 dans lequel le substrat non tissé est hydrophobe ou traité de manière hydrophobe.

3. Article (1) selon la revendication 1 ou 2 dans lequel la région de contraction chevauche la poche (13) et un sommet (15) de ladite région de contraction s'étend au-delà de ladite poche (13) dans une direction longitudinale qui coupe l'ensemble ceinture (2) et l'ensemble panneau absorbant (3).

4. Article (1) selon l'une quelconque des revendications précédentes dans lequel le substrat non tissé est conçu pour s'étendre latéralement au-delà de l'ensemble panneau absorbant (3) et dans la troisième région de taille (9).

5. Article (1) selon l'une quelconque des revendications précédentes dans lequel la poche (13) comprend un rabat qui forme une ouverture de ladite poche (13) conjointement avec une surface de la feuille supérieure faisant face à la peau.

6. Article (1) selon la revendication 5 dans lequel au moins le rabat comprend des repères, de préférence d'une couleur, de telle sorte que le rabat puisse être reconnu et soulevé pour contenir les organes génitaux masculins lorsqu'il est porté par un sujet.

7. Article (1) selon la revendication 1 dans lequel le troisième espacement est supérieur aux premier et deuxième espacements.

8. Article (1) selon l'une quelconque des revendications précédentes dans lequel les première et deuxième régions de taille (7, 8) ne chevauchent pas l'ensemble panneau absorbant (3).

9. Article (1) selon l'une quelconque des revendications précédentes dans lequel la région de contraction est positionnée sur le devant de l'article (1), de préférence uniquement sur ledit devant.

10. Article (1) selon l'une quelconque des revendications précédentes dans lequel la région de contraction est positionnée sur le devant et l'arrière de l'article (1), à condition que sur le devant, la région de contraction s'étende plus près d'une ligne médiane transversale de l'ensemble panneau absorbant (3) par rapport à l'arrière.

11. Article (1) selon l'une quelconque des revendications précédentes, se présentant sous la forme d'un sous-vêtement présentant des coutures latérales qui relient les parties avant et arrière de l'ensemble ceinture (2) l'une à l'autre.

12. Article (1) selon l'une quelconque des revendications précédentes comprenant une autre région de contraction (15) s'étendant en continu de la première et/ou la deuxième région(s) de taille (7, 8) sur le devant dudit article (1) à la première et/ou la deuxième région(s) de taille (7, 8) sur l'arrière dudit article (1) et chevauchant tout l'ensemble panneau absorbant (3).
